# EUROPEAN PATENT APPLICATION

(11) **EP 4 599 831 A1**
(43) Date of publication of application: **13.08.2025**
(21) Application number: 25156676.6
(22) Date of filing: 07.02.2025
(51) Int. Cl.: A61K 31/444, A61P 1/00, A61P 1/08

(54) **TREATMENT OF GASTROPARESIS WITH TRADIPITANT**

(30) Priority: 09.02.2024 US 202463551799 P
(71) Applicant: Vanda Pharmaceuticals Inc., Washington, DC 20037 (US)
(72) Inventor: POLYMEROPOULOS,, Mihael, H., Potomac, MD (US); SMIESZEK, Sandra, Washington, DC (US)
(74) Representative: Appleyard Lees IP LLP

(57) **Abstract**

Disclosed herein is tradipitant for use in the treatment of an individual suffering from gastroparesis or delayed gastric emptying. In an embodiment, the treatment method comprises administering to the individual tradipitant at a dose of 150-400 mg/day, wherein the individual has a genetic sequence containing a variant allele in a gene selected from the group consisting of SLC4A4, SDK2, LCLATI, SLC25A16, and CNTN1.

## Description

### CROSS REFERENCE TO RELATED APPLICATIONS

The present patent application claims the benefit of US provisional patent application no. 63/551,799, filed February 09, 2024.

### BACKGROUND OF THE INVENTION

The application relates generally to the use of NK-1 receptor antagonists for the treatment of gastroparesis, and more particularly, to the use of tradipitant for treatment of symptoms of gastroparesis including, e.g., upper abdominal pain.

Gastroparesis is a serious medical condition characterized by delayed gastric emptying in the absence of mechanical obstruction, and is associated with the symptoms of nausea, vomiting, bloating, fullness after meals, and abdominal pain, along with significant impairment of social and occupational functioning. The estimated prevalence of gastroparesis in the U.S. is over 5 million patients, many of whom remain undiagnosed. Gastroparesis affects women more frequently than men. Common etiologies include diabetes (estimated at >51.7%), post-surgical gastroparesis, and idiopathic gastroparesis (estimated at ~11.3%).

The precise underlying mechanisms leading to gastroparesis are currently poorly understood and are believed to be diverse in nature. The consensus suggests that gastroparesis arises from dysregulation of the neuromuscular control of gastric movements that result in the timely emptying of stomach contents. An innate immune dysregulation and injury to the interstitial cells of Cajal (ICC) and other components of the enteric nervous system through paracrine and oxidative stress mediators are believed to be involved in the pathogenesis of gastroparesis. Little is known about the underlying genetic risk factors for gastroparesis.

Metoclopramide was approved by the US Food and Drug Administration in 1979 for the treatment of gastroparesis. However, metoclopramide carries a black box warning and limitations of use of no more than 3 months due to potential for severe side effects. Patients are therefore faced with limited therapeutic options. Clinical guidelines recommend, in addition to metoclopramide, the off label use of different drugs including erythromycin, domperidone (not approved in the U.S.), botulinum toxin injections, gastric stimulators, and a variety of surgical procedures in an effort to achieve even temporary relief of some of the symptoms of the disease. Gastroparesis treatment represents a significant unmet medical need.

Acetylcholine and the neuropeptide, substance P, are two key stimulatory neurotransmitters of the digestive system. Substance P acts by binding to the neurokinin 1 receptor (NK-1R) at the gastric neuromuscular junction. It is believed that there is functional interplay between the acetylcholine and NK-1R systems. Gastroparesis symptoms are also associated with aberrant physiology of the vagus nerve, which constitutes the major connection between the stomach and the central nervous system. Blockade of the NK-1 receptors may have a dual and potentially therapeutic effect in gastroparesis by affecting gastric motility through local action as well as affecting nausea and vomiting via a direct effect in the brain regions responsible for nausea and vomiting.

Tradipitant is a highly potent, selective, centrally penetrating, and orally active neurokinin-1 (NK-1) receptor antagonist, depicted below as the compound of Formula I

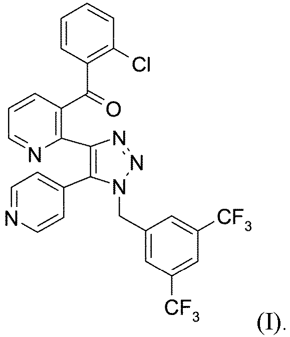

Tradipitant is disclosed in U.S. Patent No. 7,320,994, and contains six main structural components: the 3,5-bis-trifluoromethylphenyl moiety, two pyridine rings, the triazol ring, the chlorophenyl ring, and the methanone. Tradipitant is known by the chemical names, 2-[1-[[3,5-bis(trifluoromethyl)phenyl]methyl]-5-(4-pyridinyl)-1H-1,2,3-triazol-4-yl]-3-pyridinyl](2-chlorophenyl)-methanone, and {2-[1-(3,5-bistrifluoromethylbenzyl)-5-pyridin-4-yl-1H-[1,2,3]triazol-4-yl]-pyridin-3-yl}-(2-chlorophenyl)-methanone, and has also been known as LY686017 and as VLY-686. U.S. Patent 7,320,994 describes methods for using compounds including tradipitant for treating a condition associated with an excess of tachykinins, most particularly where the condition associated with an excess of tachykinins is depression and anxiety. U.S. Patent No. 7,320,994 further describes the possibility of using compounds, such as tradipitant, in other such diseases, i.e., because these compounds inhibit the physiological effects associated with an excess of tachykinins. The patent describes the usefulness of such compounds in the treatment of numerous other disorders related to tachykinin receptor activation including psychosis, schizophrenia, and other psychotic disorders; neurodegenerative disorders such as dementia, including senile dementia of the Alzheimer's type, Alzheimer's disease, AIDS-associated dementia, and Down Syndrome; demyelinating diseases such as multiple sclerosis and amyotrophic lateral sclerosis; and other neuropathological disorders, such as peripheral neuropathy, diabetic and chemotherapy-induced neuropathy, and post-herpetic and other neuralgias; acute and chronic obstructive airway diseases such as adult respiratory distress syndrome, bronchopneumonia, bronchospasm, chronic bronchitis, drivercough, and asthma; inflammatory diseases such as inflammatory bowel disease, psoriasis, fibrositis, osteoarthritis, and rheumatoid arthritis; disorders of the musculoskeletal system, such as osteoporosis; allergies such as eczema and rhinitis; hypersensitivity disorders such as poison ivy; ophthalmic diseases such as conjunctivitis, vernal conjunctivitis, and the like; cutaneous diseases such as contact dermatitis, atopic dermatitis, urticaria, and other eczematoid dermatites; addiction disorders such as alcoholism; stress-related somatic disorders; reflex sympathetic dystrophy such as shoulder/hand syndrome; dysthymic disorders; adverse immunological reactions such as rejection of transplanted tissues and disorders related to immune enhancement or suppression such as systemic lupus erythematosis; gastrointestinal disorders or diseases associated with the neuronal control of viscera such as ulcerative colitis, Crohn's disease, and irritable bowel syndrome; disorders of bladder function such as bladder detrusor hyper-reflexia and incontinence; atherosclerosis; fibrosin and collagen diseases such as scleroderma and eosinophilic fascioliasis; irritative symptoms of benign prostatic hypertrophy; disorders associated with blood pressure, such as hypertension; or disorders of blood flow caused by vasodilation and vasospastic diseases, such as angina, migraine, and Reynaud's disease; emesis, including chemotherapy-induced nausea and emesis; and pain or nociception, for example, that are attributable to or associated with any of the foregoing conditions. Finally, the patent describes that such compounds are effective in amounts to be determined, ranging from 0.001 mg/kg/day to 100 mg/kg/day.

Tradipitant is known to be therapeutically administered through a variety of routes of administration by which it is bioavailable. U.S. Patent No. 7,320,994 discloses administration of tradipitant by oral and parenteral routes, e.g., orally, by inhalation, subcutaneously, intramuscularly, intravenously, transdermally, intranasally, rectally, occularly, topically, sublingually, and buccally, with oral administration being generally preferred for treatment.

Crystalline Forms IV and V of tradipitant are disclosed in US Patent No. 7,381,826, and a process for preparing crystalline {2-[1-(3,5-bistrifluoromethylbenzyl)-5-pyridin-4-yl-1H-[1,2,3]triazol-4-yl]-pyridin-3-yl}-(2-chlorophenyl)-methanone, Form IV is disclosed in US Patents 8,772,496 and 9,708,291.

Additionally, methods of treatment of gastroparesis and gastric motility disorders are disclosed in International Patent Application Publication Nos. WO 2019/099883 and WO 2020/117811, and US Patent Application Publication No. US 2024/0102096 A1.

### BRIEF DESCRIPTION OF THE INVENTION

Various aspects of the invention disclosed herein relate to methods of treatment with tradipitant. One such method includes selecting an individual for treatment with tradipitant based on a biomarker which indicates a presence of a variant in the Solute carrier family 4 A4 (SLC4A4) gene of the individual; and administering tradipitant to the selected individual. In certain embodiments, the tradipitant may be administered at a dose of 150-400 mg/day, 150-300 mg/day, 150-200 mg/day, about 170 mg/day, or 85 mg twice daily (bid), in an immediate release form containing tradipitant and at least one pharmaceutically acceptable excipient. The immediate release form may be, e.g., a capsule or a tablet for oral administration.

In certain embodiments, selecting the individual for treatment comprises determining that the individual suffers from gastroparesis, or more particularly, from idiopathic gastroparesis. In other embodiments, the individual may suffer from diabetic gastroparesis or post-surgical gastroparesis. In certain embodiments, the selecting further comprises determining that the individual suffers from delayed gastric emptying.

In certain embodiments, the selecting further comprises determining the presence or absence of the variant in the SLC4A4 gene of the individual. This may be accomplished by obtaining or having obtained a biological sample from the patient; and performing or having performed a genotyping assay on the biological sample to determine if the individual has a SLC4A4 gene variant genotype. Performing the genotyping assay may include extracting or having extracted genomic DNA or mRNA from the biological sample, and sequencing or having sequenced SLC4A4 DNA derived from the extracted genomic DNA or from the extracted mRNA. The step of sequencing or having sequenced the DNA includes amplifying or having amplified a SLC4A4 region in the extracted genomic DNA or mRNA to prepare a DNA sample enriched in DNA from the SLC4A4 gene region; and sequencing or having sequenced the DNA sample by hybridizing the DNA sample to nucleic acid probes to determine if the patient has a SLC4A4 variant genotype.

In certain embodiments, the variant in the SLC4A4 gene that is detected in the selected individual is rs7689609-C.

According to another aspect of the invention, a method is provided, comprising selecting an individual for treatment with tradipitant based on a presence of a variant allele in a gene of the individual that is selected from the group consisting of SLC4A4, SDK2, LCLATI, SLC25A16, and CNTN1; and administering to the individual tradipitant at a dose of 150-400 mg/day.

In certain embodiments, the selecting further comprises determining that the individual suffers from gastroparesis, and that the individual suffers from a symptom of gastroparesis selected from abdominal pain and nausea, wherein the dose of tradipitant is effective to treat the one or more symptom of gastroparesis.

According to another aspect of the invention, tradipitant is provided for use according to any preceding method.

These and other aspects, advantages and salient features of the invention will become apparent from the following detailed description, which, when taken in conjunction with the annexed drawings, disclose embodiments of the invention.

### DETAILED DESCRIPTION OF THE INVENTION

At least one embodiment of the present invention is described below in reference to its application in connection with the use of tradipitant for the treatment of gastroparesis. Although some embodiments of the invention are illustrated relative to a specific disorder, i.e., gastroparesis, it is understood that the teachings are equally applicable to symptoms associated with gastroparesis, which may include nausea, vomiting, early satiety, postprandial fullness, dyspepsia (indigestion), functional dyspepsia, bloating, and abdominal pain. The teachings are further applicable to both individuals who have and who have not been diagnosed with gastroparesis, as well as those suffering from delayed gastric emptying. Further, the individual may suffer from idiopathic gastroparesis, post-operative gastroparesis, or diabetic gastroparesis.

Individuals suffering from gastroparesis can be treated by orally administering tradipitant in an effective amount, i.e., an effective dose. As used herein, the term "effective amount" or "effective dose" of tradipitant refers to an amount or dose that is effective in treating the disorders described herein, and may refer to an amount in conjunction with a dosing frequency required to achieve therapeutic plasma concentrations of tradipitant in plasma, e.g., at least about 100 ng/mL, e.g., 125 ng/mL or greater, 150 ng/mL or greater, 175 ng/mL or greater, 200 ng/mL or greater, or 225 ng/mL or greater. Such plasma concentration levels can be achieved, e.g., by orally administering to the patient tradipitant in a solid immediate release form comprising one or more pharmaceutically acceptable excipients and tradipitant in an amount of, e.g., 100 to 400 mg/day, 100 to 300 mg/day, 100 to 200 mg/day, or about 85 to 170 mg/day, which may be administered as, e.g., 50 to 200 mg bid, 50 to 150 mg bid, 50 to 100 mg bid, or about 85 mg bid, or about 150 to 400 mg/day, 150 to 300 mg/day, or about 150 to 200 mg/day, which may be administered as, e.g., 75 to 200 mg bid, 75 to 150 mg bid, or 75 to 100 mg bid, or about 85 mg bid, as described in PCT publication WO 2016/141341 A1. With regard to dosing, "qd" refers to dosing once per day; "bid" dosing typically means dosing once in the morning and once in the evening, generally no less than about 8 hours or more than about 16 hours apart, e.g., 10 to 14 hours or 12 hours (Q12H).

As used herein, the terms "patient," "subject," and "individual" refer to human beings, as well as companion animals (e.g., dogs and cats) and other domesticated animals (e.g., horses, cattle, and sheep). It will be understood that the most preferred patient is a human being.

The invention further relates to the treatment of gastroparesis or delayed gastric emptying, and symptoms thereof, either prophylactically or therapeutically. Further, the terms "treatment" and "treating" are intended to refer to all processes wherein there may be a slowing, interrupting, arresting, controlling, or stopping of the progression of the disorders described herein, and is intended to include prophylactic treatment of such disorders, but does not necessarily indicate a total elimination of all disorder symptoms.

In one embodiment, a method is provided for treating an individual as described herein. According to the method, the individual may be selected for treatment with tradipitant based on a determination that the individual's genotype includes a genetic variant in one or more genes that is associated with increased frequency, intensity, or the like of a symptom of gastroparesis. For example, the variant may be in one or more of the SLC4A4, SDK2, LCLAT1, SLC25A16, and CNTN1 genes. The variants may be associated with, e.g., abdominal pain, e.g., upper abdominal pain, nausea, or another symptom of gastroparesis as described herein at baseline. The variant may be detected in a biological sample taken from the patient. The individual may further be selected on the basis of a medical professional's conclusion that the individual suffers from gastroparesis, particularly idiopathic gastroparesis, or delayed gastric emptying.

As noted, the individual selection process may include determining the presence or absence of the variant in the gene of interest, e.g., SLC4A4, SDK2, LCLAT1, SLC25A16, and CNTN1, in the individual. In certain embodiments, such a determination may be made by obtaining or having obtained a biological sample from the patient; and performing or having performed a genotyping assay on the biological sample to determine if the individual has a variant genotype in SLC4A4, SDK2, LCLAT1, SLC25A16, or CNTN1. In this context, "obtaining" may refer to collecting or acquiring a biological sample from the patient, while "having obtained" may refer to referring, instructing, or otherwise causing another individual, e.g., a medical or healthcare professional, to perform the obtaining. "Having obtained" may also refer to having previously caused the obtaining to have been performed, e.g., where an assay that identifies the individual's SLC4A4 genotype has been performed in the past, and the result may be reviewed in the individual's medical records. Similarly, in this context, "performing the genotyping assay" may refer to physically performing the steps to examine the individual's DNA using a genotyping assay, while "having performed" may refer to referring, instructing, or otherwise causing another individual, e.g., a medical or healthcare professional, to carry out the performing. The expression, "having performed" may also refer to having previously caused the performance of the assay. Performing (or having performed) the assay may include the steps of extracting or having extracted genomic DNA or mRNA from the biological sample, and sequencing or having sequenced SLC4A4 DNA derived from the extracted genomic DNA or from the extracted mRNA. The sequencing (or having sequenced) step may further comprise amplifying or having amplified a SLC4A4 region in the extracted genomic DNA or mRNA to prepare a DNA sample enriched in DNA from the SLC4A4 gene region; and sequencing (or having sequenced) the DNA sample by hybridizing the DNA sample to nucleic acid probes to determine if the patient has a SLC4A4 variant genotype.

In certain embodiments, the gene of interest may be SLC4A4, and the SLC4A4 variant detected in a selected individual may be rs7689609-C. In other embodiments, variants may be detected within SDK2 and LCLAT1, SLC25A16, and CNTN1 in the selected individual. Other variants will be known and understood by one of skill in the art.

In the event that the individual has a wild type genotype at, e.g., the SLC4A4 locus, and is not a carrier of a SLC4A4 variant as described herein, the individual may not be selected for treatment, or may be selected for a treatment that differs in one or more of the active pharmaceutical ingredient, the dose selected, or other features of the treatment regimen. On the other hand, in the event that the individual has a SLC4A4 variant genotype, i.e. a non-wild type genotype at the SLC4A4 locus, e.g., rs7689609-C, the individual may be selected for treatment according to further steps of the method described herein.

Similarly, in the event that the individual has a wild type genotype at, e.g., the SLC4A4, SDK2, LCLAT1, SLC25A16, and CNTN1 loci, and is not a carrier of a SLC4A4, SDK2, LCLAT1, SLC25A16, or CNTN1 variant as described herein, the individual may not be selected for treatment, or may be selected for a treatment that differs in one or more of the active pharmaceutical ingredient, the dose selected, or other features of the treatment regimen. On the other hand, in the event that the individual has a SLC4A4 variant genotype, i.e. a non-wild type genotype at the SLC4A4 locus, e.g., rs7689609-C, and/or a SDK2, LCLAT1, SLC25A16, and/or CNTN1 variant genotype, the individual may be selected for treatment according to further steps of the method described herein.

Following selection of the individual for treatment as described herein, the method may further include administering to the selected individual tradipitant at a dose effective to treat the individual's symptoms of gastroparesis, e.g., upper abdominal pain. Exemplary effective doses may be, e.g., 150-400 mg/day, 100-400 mg/day, 150 to 300 mg/day, 100-300 mg/day, 150 to 200 mg/day, 100-200 mg/day, or about 170 mg/day. The dosage amount may be administered as, e.g., 75 to 200 mg bid, 50-200 mg bid, 75 to 150 mg bid, 50-150 mg bid, 75 to 100 mg bid, 50-100 mg bid, or about 85 mg bid. As described herein, the dose administered may be sufficient to achieve and maintain a plasma concentration level of tradipitant in the individual of at least about 100 ng/mL, e.g., 125 ng/mL or greater, 150 ng/mL or greater, 175 ng/mL or greater, 200 ng/mL or greater, or 225 ng/mL or greater.

### Example 1: Phenome-wide association study

Whole genome sequencing (WGS) samples are obtained from adult gastroparesis (GP) patients with a diagnosis of diabetic or idiopathic gastroparesis, having evidence of delayed gastric emptying, and moderate to severe nausea, a daily average nausea score of greater than 2.5, at least one episode of vomiting, a Patient Assessment of Gastrointestinal Disorders-Symptom Severity Index (PAGI-SYM) nausea score of greater than or equal to 2 at screening, and controlled blood glucose levels with HbA1c < 10.

A phenome-wide association study (PheWAS) is performed, and identifies that some of the gastrointestinal disease-related variants colocalize with cis-regulatory elements of the electrical slow wave associated gene SLC4A4 in a human model of pacemaker interstitial cells of cajal (ICC). The Na⁺/HCO₃⁻ cotransporter SLC4A4 is selectively expressed in pacemaker ICCs and contributes to slow waves.

The rs7689609-C variant within SLC4A4 demonstrates a higher allele frequency in gastroparesis cases compared to a control population (0.63 from the Genome Aggregation Database (gnomAD) total, and 0.72 in the gastroparesis (GP) randomized samples).

The effect of the rs7689609-C variant within SLC4A4 is analyzed in the set of WGS samples. A linear regression model is used to study the genetic additive effect of rs7689609-C on upper abdominal pain, adjusting for age, sex, BMI, PC1 and PC2. A statistically significant association is observed between the SLC4A4 rs7689609-C variant and baseline upper abdominal pain in the randomized set (beta=0.412, p=0.016, n=168). This effect persists when analyzed together with a larger set of open-label studies (beta=0.311, p=0.009, n=446). The effect of the variant is particularly pronounced in females (beta=0.350, p=0.009, n=355), and particularly in females with idiopathic gastroparesis.

Each additional copy of the SLC4A4 rs7689609-C allele increases the upper abdominal pain frequency. At 12 weeks of the study, the SLC4A4 rs7689609-C variant is associated with upper abdominal pain (p=0.018) and nausea (p=0.069). Abdominal pain is moderately correlated with bloating and fullness. It is hypothesized that the SLC4A4 rs7689609-C variant is a relevant marker of the inflammatory subtypes of manifestation of gastroparesis-related abdominal pain.

In addition, a genome-wide association analysis is performed on a baseline abdominal pain severity scale adjusting for age, sex, BMI and PC1-3. Variants within SDK2, LCLAT1, SLC25A16, and CNTN1 are delineated as reaching genome-wide significance threshold. CNTN1 is known to be associated with autoimmune neuropathy and neuropathic pain, while SLC25A16 has been described in association with gastrointestinal system disease.

The SLC4A4 rs7689609-C variant may be a relevant marker of the inflammatory subtypes of manifestation of gastroparesis-related abdominal pain. Further understanding of the SDK2, LCLAT1, SLC25A16, and CNTN1 loci in association with symptoms may further help delineate mechanisms responsible for etiology of subtypes gastroparesis.

### Embodiments

Embodiments of the present disclosure may include the following features:
Item 1. A method comprising: selecting an individual for treatment with tradipitant based on a presence of a variant allele in the SLC4A4 gene of the individual; and administering to the individual tradipitant at a dose of 150-400 mg/day.
Item 2. The method of item 1, wherein the dose of tradipitant is 150-300 mg/day.
Item 3. The method of item 2, wherein the dose of tradipitant is 150-200 mg/day.
Item 4. The method of item 3, wherein the dose of tradipitant is 170 mg/day.
Item 5. The method of item 4, wherein the dose of tradipitant is 85 mg twice daily (bid).
Item 6. The method of any one of item 1-5, wherein the variant allele in the SLC4A4 gene is rs7689609-C.
Item 7. The method of item 6, wherein the individual has a SLC4A4 genotype that includes one rs7689609-C allele.
Item 8. The method of item 6, wherein the individual has a SLC4A4 genotype that includes two rs7689609-C alleles.
Item 9. The method of any one of items 1-8, wherein the individual suffers from one or both of abdominal pain and nausea.
Item 10. The method of item 9, wherein the dose of tradipitant is effective to treat the abdominal pain or the nausea.
Item 11. The method of any one of items 1-10, wherein the selecting further comprises determining that the individual suffers from gastroparesis.
Item 12. The method of item 11, wherein the gastroparesis further comprises idiopathic gastroparesis.
Item 13. The method of item 11, wherein the gastroparesis further comprises diabetic gastroparesis.
Item 14. The method of item 11, wherein the gastroparesis further comprises post-operative gastroparesis.
Item 15. The method of any one of item 1-14, wherein the selecting further comprises: determining the presence or absence of the variant in the SLC4A4 gene of the individual by: obtaining or having obtained a biological sample from the patient; and performing or having performed a genotyping assay on the biological sample to determine if the individual has a SLC4A4 gene variant genotype.
Item 16. The method of item 15, wherein the performing or having performed the genotyping assay step comprises: extracting or having extracted genomic DNA or mRNA from the biological sample, and sequencing or having sequenced SLC4A4 DNA derived from the extracted genomic DNA or from the extracted mRNA, wherein the sequencing or having sequenced step further comprises: amplifying or having amplified a SLC4A4 region in the extracted genomic DNA or mRNA to prepare a DNA sample enriched in DNA from the SLC4A4 gene region; and sequencing or having sequenced the DNA sample by hybridizing the DNA sample to nucleic acid probes to determine if the patient has a SLC4A4 variant genotype.
Item 17. A method comprising: selecting an individual for treatment with tradipitant based on a presence of a variant allele in a gene of the individual that is selected from the group consisting of SLC4A4, SDK2, LCLATI, SLC25A16, and CNTN1; and administering to the individual tradipitant at a dose of 150-400 mg/day.
Item 18. The method of item 17, wherein the selecting further comprises determining that the individual suffers from gastroparesis.
Item 19. The method of item 18, wherein the individual suffers from a symptom of gastroparesis selected from abdominal pain and nausea, and wherein the dose of tradipitant is effective to treat the one or more symptom of gastroparesis.
Item 20. Tradipitant for use according to any preceding method.

While various embodiments are described herein, it will be appreciated from the specification that various combinations of elements, variations or improvements therein may be made by those skilled in the art, and are within the scope of the invention. In addition, many modifications may be made to adapt a particular situation or material to the teachings of the invention without departing from essential scope thereof. Therefore, it is intended that the invention not be limited to the particular embodiment disclosed, but that the invention will include all embodiments falling within the scope of the appended claims.

## Claims

1. Tradipitant or a pharmaceutically acceptable salt thereof, for use in a method for treating a patient suffering from gastroparesis, the method comprising:
administering to the patient tradipitant at a dose of 150-400 mg/day,
wherein the patient has a genetic sequence containing a variant allele in a gene selected from the group consisting of SLC4A4, SDK2, LCLATI, SLC25A16, and CNTN1.

2. Tradipitant or the pharmaceutically acceptable salt of claim 1, wherein the dose of tradipitant is 150-300 mg/day.

3. Tradipitant or the pharmaceutically acceptable salt of claim 2, wherein the dose of tradipitant is 150-200 mg/day.

4. Tradipitant or the pharmaceutically acceptable salt of claim 3, wherein the dose of tradipitant is 170 mg/day.

5. Tradipitant or the pharmaceutically acceptable salt of claim 4, wherein the dose of tradipitant is 170 mg/day given as 85 mg twice daily (bid).

6. Tradipitant or the pharmaceutically acceptable salt of any one of claims 1-5, wherein the variant allele is in the SLC4A4 gene.

7. Tradipitant or the pharmaceutically acceptable salt of claim 6, wherein the variant allele in the SLC4A4 gene is rs7689609-C.

8. Tradipitant or the pharmaceutically acceptable salt of claim 7, wherein the patient has a SLC4A4 genotype that includes one rs7689609-C allele.

9. Tradipitant or the pharmaceutically acceptable salt of claim 7, wherein the patient has a SLC4A4 genotype that includes two rs7689609-C alleles.

10. Tradipitant or the pharmaceutically acceptable salt of any one of claims 1-9, wherein the patient suffers from one or both of abdominal pain and nausea.

11. Tradipitant or the pharmaceutically acceptable salt of claim 10, wherein the dose of tradipitant is effective to treat the abdominal pain or the nausea.

12. Tradipitant or the pharmaceutically acceptable salt of claim 11, wherein the gastroparesis further comprises idiopathic gastroparesis.

13. Tradipitant or the pharmaceutically acceptable salt of claim 11, wherein the gastroparesis further comprises diabetic gastroparesis.

14. Tradipitant or the pharmaceutically acceptable salt of claim 11, wherein the gastroparesis further comprises post-operative gastroparesis.
